# EUROPÄISCHE PATENTSCHRIFT

(11) **EP 2 849 718 B1**
(45) Veröffentlichungstag und Bekanntmachung des Hinweises auf die Patenterteilung: **01.02.2017**
(21) Anmeldenummer: 13718558.3
(22) Anmeldetag: 18.04.2013
(51) Int. Cl.: A61K 8/34, A61K 8/33, A61Q 19/00, A61Q 11/00, A61Q 13/00, A61K 31/015, A23G 9/32, A61K 31/045, A61K 31/11

(54) **MISCHUNGEN MIT VERBESSERTER KÜHLWIRKUNG**
MIXTURES HAVING IMPROVED COOLING EFFECT
MÉLANGES À EFFET RAFRAÎCHISSANT AMÉLIORÉ

(30) Priorität: 16.05.2012 EP 12003859
(43) Veröffentlichungstag der Anmeldung: 25.03.2015
(73) Patentinhaber: Symrise AG, 37603 Holzminden (DE)
(72) Erfinder: LAGES, Rita, 37619 Bodenwerder (DE); LOGES, Hubert, 37671 Höxter (DE); MACHINEK, Arnold, 37603 Holzminden (DE); NEUENDORFF, Gabriele, 37603 Holzminden (DE); DILK, Erich, 37603 Holzminden (DE); OERTLING, Heiko, CH-1012 Lausanne (CH)
(74) Vertreter: Copsey, Timothy Graham
(86) Internationale Anmeldenummer: PCT/EP2013/058118
(87) Internationale Veröffentlichungsnummer: WO 2013/171018

(56) Entgegenhaltungen:
- EP-A2- 0 988 852
- EP-A2- 1 332 772
- WO-A1-00/01321
- WO-A1-00/62737
- WO-A2-2008/107137
- US-A- 3 879 425
- US-A- 3 922 237
- US-A1- 2011 195 042

## Beschreibung

### Gebiet der Erfindung

Die Erfindung befindet sich auf dem Gebiet Stoffe mit kühlender sensorischer Wirkung und betrifft Mischungen mit verbesserter Kühlwirkung und Zubereitungen, die diese Mischungen enthalten.

### Stand der Technik

Um dem Bedarf des Verbrauchers nach immer neuen Geruchs- und Geschmackserlebnissen nachzukommen, besteht in der Aromen- und Geschmackstoffindustrie ein großer Bedarf an Stoffen, die herausragende sensorische (d.h. mit den Sinnen wahrnehmbare) Eigenschaften besitzen und mit denen sich bemerkenswerte neuartige Effekte erzielen lassen. Dabei können neben den reinen geruchlichen und geschmacklichen Eigenschaften weitere zusätzliche Eigenschaften von Bedeutung sein, so z.B. dass die Geruchs- und Geschmacksempfindungen gehemmt oder verstärkt werden.

Aroma- bzw. Geschmacksstoffkompositionen mit Kühlwirkung verleihen z.B. Mundhygieneprodukten, wie Zahnpasten und Mundwässern, und Süßwaren wie Bonbons und Kaugummis, ihren typischen, frischen und als angenehm empfundenen Geschmack.

Stoffe, die in großem Umfang für die Herstellung solcher Aroma-, bzw. Geschmacksstoffkompositionen mit Kühlwirkung verwendet werden, sind beispielsweise Eucalyptol (1,8-Cineol) und Menthol. Die Verwendung dieser Stoffe ist jedoch mit einigen Nachteilen behaftet. So zeigt das Eucalyptol neben seiner Kühlwirkung einen sehr starken medizinischen Eigengeschmack, der von vielen Konsumenten als abstoßend empfunden wird, besonders wenn das Eucalyptol in höheren Dosierungen verwendet wird. Bei der Verwendung von Menthol setzt die Kühlwirkung mit einer gewissen Verzögerung ein, und bei höheren Dosierungen entwickelt das Menthol einen bitteren, scharfen und stechenden Eigengeschmack, der eine eher unangenehme Wirkung hat.

Die Aufgabe der vorliegenden Erfindung hat somit darin bestanden, Formulierungen bereitzustellen, die eine verbesserte physiologische Kühlwirkung aufweisen und dabei geringe oder verringerte unerwünschte Eigenschaften besitzen, d.h. zusätzlich auch die geschmackliche Wahrnehmung der Kühlstoffe verbessert.

### Beschreibung der Erfindung

Gegenstand der Erfindung sind Mischungen, enthaltend

### (a) mindestens ein Phenylalkenal der Formel (I)

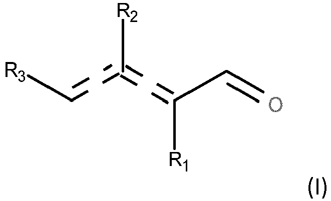

in der R₁ und R₂ unabhängig voneinander Wasserstoff, einen Methyl- oder Phenylrest darstellen, R₃ für Wasserstoff, einen Phenyl-, einen Alkenyl- oder einen linearen oder verzweigten Alkylrest mit 1 bis 5 C-Atomen stehen, und die gestrichelten Doppellinien unabhängig voneinander eine Einfachbindung oder Doppelbindung darstellen, und

### (b) mindestens einen physiologischen Kühlwirkstoff.

Überraschenderweise wurde gefunden, dass die erfindungsgemäße Mischung eine verstärkte Kühlwirkung aufweist, ohne die unerwünschten Eigenschaften der physiologischen Kühlwirkstoffe, wie Stechen, Brennen oder Bitterkeit zu verstärken. Insbesondere kann bei Verstärkung der Kühlwirkung der physiologischen Kühlstoffe sogar eine Verminderung des Stechens, Brennens oder der Bitterkeit erzielt werden.

Dieser Effekt war nicht vorherzusehen, da im Stand der Technik beschrieben wird, dass 2-Phenyl-but-2-enal gemäß DE1921560 gerade deswegen verwendet wird, um Lebensmitteln eine stechende Kakaonote zu verleihen. Ebenso überraschend ist, dass die erfindungsgemäßen Mischungen keine Kakao-, Nuss-, Kaffee- bzw. Fischnoten besitzen.

### Phenylalkenale

Phenylalkenale (Komponente a) stellen bekannte Stoffe dar, die nach den einschlägigen Verfahren der organischen Chemie erhältlich sind und die beispielsweise von Maruoka et al. in JACS 11, S. 7922-7924 (1988**)** beschrieben werden

2-Phenyl-but-2-enal findet bereits Verwendung in verschiedensten Zusammensetzungen. So wird in WO 2010 146258 A die Verwendung von 2-Phenyl-but-2-enal in Deokompositionen und in WO 2008 149102 A als Konservierungsstoff beschrieben. In WO 2008 049581 A wird 2-Phenyl-but-2-enal in Milcharomen und in WO 2008 011742 A als Geschmacksvorstufe für Lebensmittel offenbart. JP 2006 121958 A**,** JP 2006 025706 A**,** JP 200 020526 A**,** JP 2004 135522 A sowie DE 1921560 A offenbaren 2-Phenyl-but-2-enal zur Verbesserung von Kakao-, Nuss-, Kaffee- und Fischaromen.

Besonders bevorzugt sind im Sinne der Erfindung Phenylalkenale der Formel (I) in der R₁ und R₂ verschieden sind und Wasserstoff oder Phenyl bedeuten, R₃ für Wasserstoff, eine Alkenyl- oder eine lineare oder verzweigte Alkylgruppe mit 1 bis 5 C-Atomen steht, und die gestrichelten Doppellinien unabhängig voneinander eine Einfachbindung oder Doppelbindung darstellen.

In einer ganz bevorzugten Ausführungsform der Erfindung wird das Phenylalkenal nach Formel (I) ausgewählt aus der Gruppe, die gebildet wird von 2-Phenyl-but-2-enal, 2-Phenyl-pent-2-enal, 3-Phenyl-pent-4-enal, 2-Phenyl-pent-4-enal und deren Mischungen.

Die Formeln und CAS-Nr. der besonders bevorzugten Verbindungen sind in der folgenden Tabelle I aufgeführt:

**Tabelle I**

| CAS-Nummern und Formeln der bevorzugten Ausführungsformen für die Komponente a | | |
|---|---|---|
| **Name** | **CAS-Nr.** | **Formel** |
| 2-Phenyl-but-2-enal | 4411-89-6 | 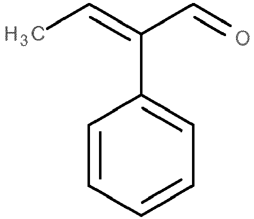 |
| | | Formel Ia |
| 2-Phenyl-pent-2-enal | 3491-63-2 | 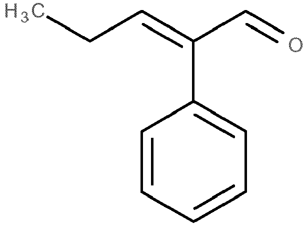 |
| | | Formel Ib |
| 3-Phenyl-pent-4-enal | 939-21-9 | 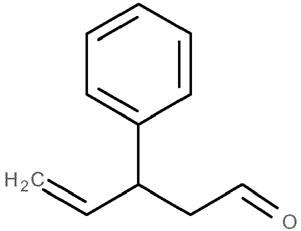 |
| | | Formel Ic |
| 2-Phenyl-pent-4-enal | CAS 24401-36-3 | 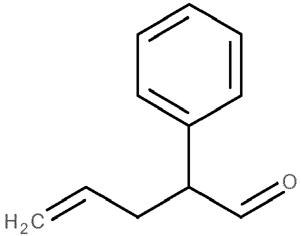 |
| | | Formel Id |

### Kühlstoffe

Physiologische Kühlstoffe (Komponente b), die im Sinne der vorliegenden Erfindung geeignet sind, stellen überwiegen Menthol oder Mentholverbindungen dar. Physiologisch bedeutet in diesem Zusammenhang, dass sie auch für die orale Anwendung zugelassen sind.

Diese umfassen - neben dem Grundkörper Menthol selbst - Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter der Stoffe, die die Komponente (b) bilden, stellt das Monomenthyl Succinate (FEMA GRAS 3810) dar, das als Stoff bereits 1963 von Brown & Williamson Tobacco Corp. patentiert worden **(**US 3,111,127**)** und als Kühlmittel Gegenstand der Schutzrechte US 5,725,865 und 5,843,466 (V.Mane Fils**)** ist. Sowohl das Succinat als auch das analoge Monomenthyl Glutarate (FEMA GRAS 4006) stellen wichtige Vertreter von Monomenthylestern auf Basis von Di- und Polycarbonsäuren dar:

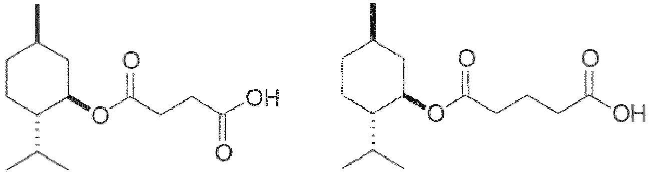

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten:

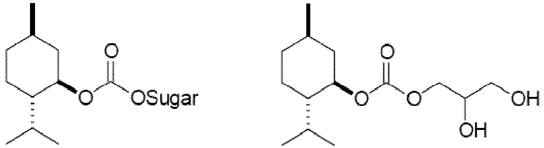

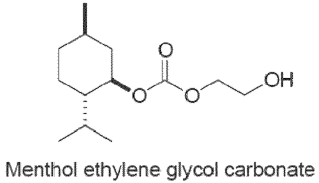

Die Verwendung derartiger Stoffe als Kühlstoff für Zigaretten ist beispielsweise Gegenstand der Druckschrift US 3,419,543 (Mold et al.) aus dem Jahre 1968; die Anwendung als physiologisches Kühlmittel wird in DE 4226043 A1 (H&R) beansprucht.

Im Sinne der Erfindung bevorzugt sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird.

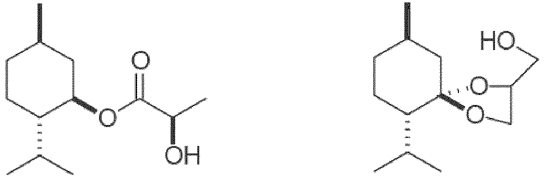

Erstere Struktur wird durch Veresterung von Milchsäure mit Menthol, letztere durch Acetalisierung von Menthon mit Glycerin gewonnen (vgl. DE 2608226 A1**,** H&R). In diese Gruppe von Verbindungen gehört auch das 3-(I-Menthoxy)-1,2,propandiol, das auch als Cooling Agent 10 bekannt ist (FEMA GRAS 3784, vgl. US 6,328,982**,** TIC), sowie das 3-(I-Menthoxy)-2-methyl-1,2,propandiol (FEMA GRAS 3849), das über eine zusätzliche Methylgruppe verfügt.

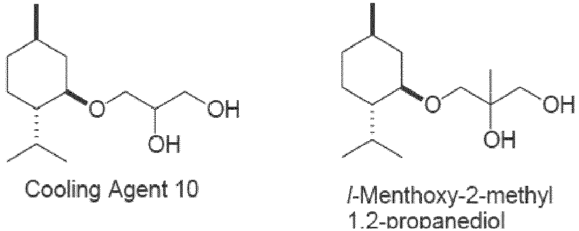

Die Herstellung des 3-(I-Menthoxy)-1,2,propandiol erfolgt beispielsweise ausgehend von Menthol nach dem folgenden Schema (vgl. US 4,459,425**,** Takagaso):

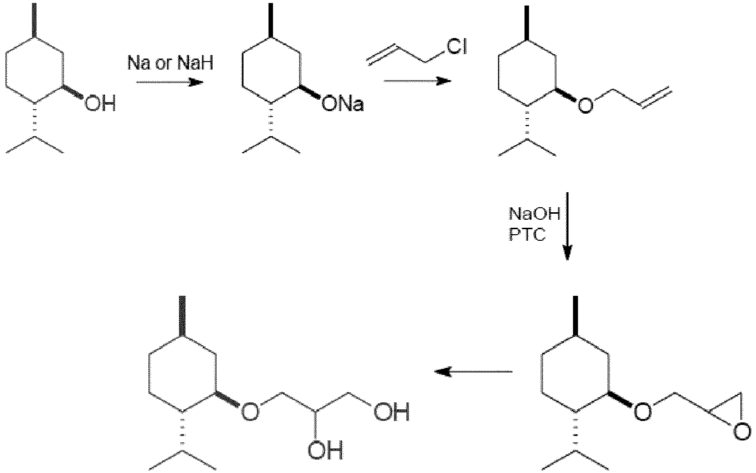

Alternative Routen, bei denen in der ersten Stufe Menthol mit Epichlorhydrin umgesetzt wird, wird in US 6,407,293 und US 6,515,188 (Takagaso) beschrieben. Im Folgenden wird eine Übersicht der bevorzugten Mentholverbindungen gegeben, die sich durch eine CO-Bindung auszeichnen:

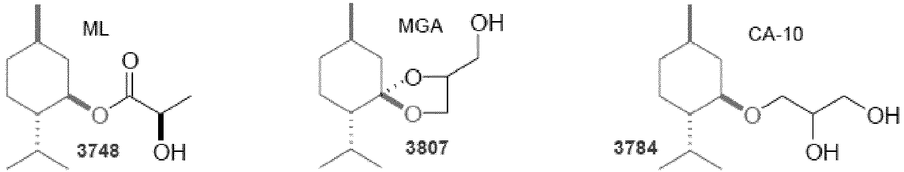

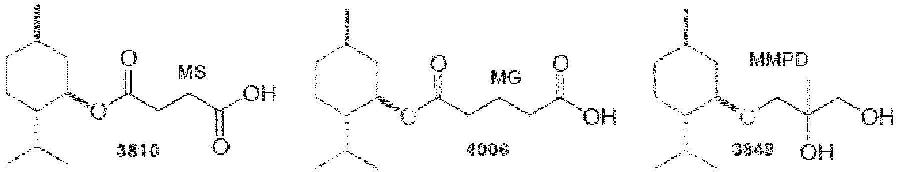

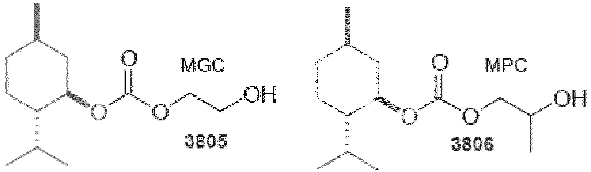

Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat^{®} MGA, Frescolat^{®} ML, Frecolat^{®} MGC und Frescolat^{®} MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern im Sinne der Erfindung eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die ebenfalls im Sinne der Erfindung bevorzugten Spezies WS-3, WS-4, WS-5, WS-12, WS-14 und WS-30. Die beiden nachfolgenden Schaubilder zeigen die Synthesewege auf:

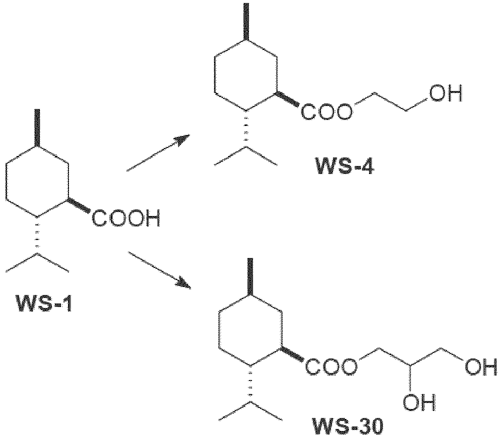

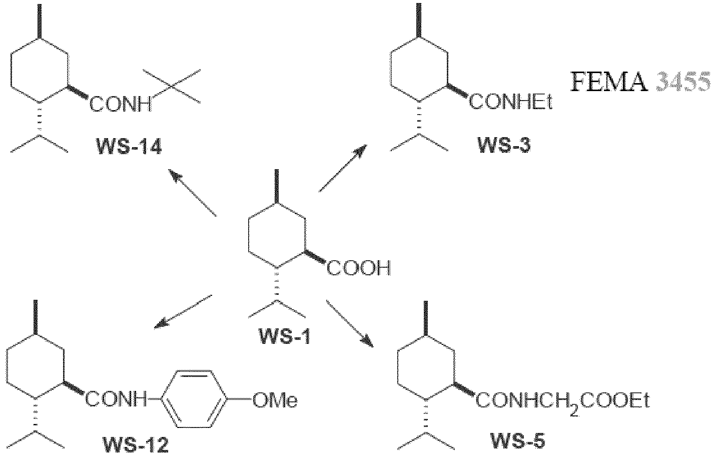

Die sich von WS-1 ableitenden Ester werden beispielsweise in US 4,157,384**,** die entsprechenden N-substituierten Amide in J. Soc. Cosmet. Chem. S. 185-200 (1978**)** beschrieben.

Besonders bevorzugt sind Zubereitungen, die als Komponente (b) Kühlstoffe enthalten, die ausgewählt sind aus der Gruppe, die gebildet wird von Menthol, L-Menthol, Menthonglycerinacetal, Menthyllactat, substituierten Menthan-3-carbonsäureamiden, Menthan-3-carbonsäure-N-ethylamid, 2-Isopropyl-N,2,3-trimethylbutanamid, substituierten Cyclohexancarbonsäureamiden, 3-Menthoxy-1,2-propandiol, 2-Hydroxyethylmenthylcarbonat, 2-Hydroxypropylmenthylcarbonat, N-Acetylglycinmenthylester, Menthylhydroxycarbonsäureestern, . Menthyl-3-hydroxybutyrat, Menthylmonosuccinat, 2-Mercaptocyclodecanon, Menthyl-2-pyrrolidin-5-oncarboxylat und deren Mischungen.

### Weitere Zusatzstoffe

In einer bevorzugten Ausführungsform der vorliegenden Erfindung können die Mischungen des Weiteren als optionale Komponenten
(c) mindestens einen Aromastoff und/oder
(d) mindestens ein Lösungsmittel
enthalten.

### 1. Aromastoffe

Die erfindungsgemäßen Mischungen können als optionale Komponente © einen oder mehrere Aromastoffe enthalten. Typische Beispiele umfassen: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Vorzugsweise sind die Aromen jedoch ausgewählt aus der Gruppe, die gebildet wird von Anethol, Menthon, Isomenthon, Menthylacetat, Menthofuran, Mintlacton, Eucalyptol, Limonen, Eugenol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Hexanol, Hexanal, cis-3-Hexenol, Linalool, alpha-Terpineol, cis und trans Carvylacetat, p-Cymol, Damascenon, Damascone, Rosenoxid, Dimethylsulfid, Fenchol, Acetaldehyddiethylacetal, cis-4-Heptenal, Isobutyraldehyd, Isovaleraldehyd, cis-Jasmon, Anisaldehyd, Methylsalicylat, Myrtenylacetat, 2-Phenylethylalkohol, 2-Phenylethylisobutyrat, 2-Phenylethylisovalerat, Zimtaldehyd, Geraniol, Nerol und deren Mischungen.

### 2. Lösungsmittel

Des Weiteren können die Mischungen als optionale Komponente (d) Lösungsmittel enthalten. Hierbei kommen insbesondere solche in Frage, die ausgewählt sind aus der Gruppe, die gebildet wird von Ethylalkohol, 1,2-Propylenglycol, Triacetin, Benzylakohol und fetten Ölen und deren Mischungen.

### Mischungen

Die erfindungsgemäßen Mischungen können die einzelnen Komponenten in folgenden Mengen enthalten:
(a) etwa 1 bis etwa 99 Gew.-%, vorzugsweise etwa 20 bis etwa 80 Gew.-% und insbesondere etwa 30 bis etwa 70 Gew.-% Phenylalkenale der Formel (I) und
(b) etwa 99 bis etwa 1 Gew.-%, vorzugsweise etwa 80 bis etwa 20 Gew.-% und insbesondere etwa 70 bis etwa 30 Gew.-% Kühlstoffe,
mit der Maßgabe, dass sich die Mengenangaben zu 100 Gew.-% ergänzen.

Die optionalen Komponenten (c) und (d) können bezogen auf die Mischung (a+b) jeweils in Mengen von jeweils etwa 0,1 bis etwa 70 Gew.-%, vorzugsweise etwa 1 bis etwa 60 Gew.-% und insbesondere etwa 5 bis etwa 50 Gew.-% zugegeben werden.

### Gewerbliche Anwendbarkeit

### Kosmetische und/oder pharmazeutische Mittel einschließlich Mund- und Zahnpflegemittel

Ein weiterer Gegenstand der vorliegenden Erfindung betrifft kosmetische und/oder pharmazeutische Zubereitungen sowie speziell Mund- und Zahnpflegemittel, die die erfindungsgemäßen Mischungen vorzugsweise in Mengen von etwa 0,0001 bis etwa 20 Gew.-%, besonders bevorzugt etwa 0,0005 bis etwa 10 Gew.-% und insbesondere etwa 0,001 bis etwa 5 Gew.-% und weiter besonders bevorzugt 0,001 bis etwa 1 Gew.-% enthalten.

Diese Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen aufweisen.

### 1. Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### 2. Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### 3. Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia^{®} SP von Cognis;
- Polyalkylenglycole sowie
- Glycerincarbonat.

Im Folgenden werden besonders geeignete Emulgatoren näher erläutert:
(i) **Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.
(ii) **Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.
(iii) **Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
(iv) **Sorbitanester.** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.
(v) **Polyglycerinester.** Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.
(vi) **Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.
(vii) **Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### 4. Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### 5. Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### 6. Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und - diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### 7. Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### 8. Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### 9. Silikonverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### 10. UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan^{®} AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000, Eusolex^{®} T, Eusolex^{®} T-ECO, Eusolex^{®} T-S, Eusolex^{®} T-Aqua, Eusolex^{®} T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE^{®} oder Z-COTE HP1^{®} verwendet.

### 11. Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### 12. Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### 13. Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.
(i) **Keimhemmende Mittel.** Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.
(ii) **Enzyminhibitoren.** Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.
(iii) **Geruchsabsorber.** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.
(iv) **Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
   - adstringierende Wirkstoffe,
   - Ölkomponenten,
   - nichtionische Emulgatoren,
   - Coemulgatoren,
   - Konsistenzgeber,
   - Hilfsstoffe wie z. B. Verdicker oder Komplexierungsmittel und/oder
   - nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### 14. Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### 15. Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival^{®} (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### 16. Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### 17. Insektenrepellentien

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### 18. Inhaltsstoffe für Mund- und Zahnpflegemittel

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxidtrihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol^{®}-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam^{®}, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

### 19. Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### 20. Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

### 21. Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-%-bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Nahrungsmittel

Ein weiterer Gegenstand der Erfindung betrifft Nahrungsmittel, die die erfindungsgemäßen Mischungen vorzugsweise in Mengen von etwa 0,5 bis etwa 20 Gew.-%, besonders bevorzugt etwa 1 bis etwa 15 Gew.-% und insbesondere etwa 2 bis etwa 10 Gew.-% enthalten.

Vorzugsweise handelt es sich bei den oralen Zubereitung um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

### Kaugummis

Die erfindungsgemäßen Mischungen können auch in Kaugummizubereitungen Eingang finden. Kaugummis können dann je nach Zusammensetzung als Mund- und Zahnpflegemittel (medizinische Kaugummis) oder als Genussmittel und damit als Teilmenge der Nahrungsmittel aufgefasst werden. Die Einsatzmenge der erfindungsgemäßen Mischungen kann dabei zwischen etwa 0,5 und etwa 5 Gew.-%, vorzugsweise etwa 1 bis 3 etwa 3 Gew.-% und insbesondere etwa 1,5 bis 2,5 Gew.-% liegen.

Kaugummis enthalten typischerweise eine wasserunlösliche und eine wasserlösliche Komponente. Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### Aroma- und Parfümzubereitungen

Ein weiterer Gegenstand der Erfindung betrifft Aroma- und Parfümzubereitungen, die die erfindungsgemäßen Mischungen vorzugsweise in Mengen von etwa 0,005 bis etwa 50 Gew.-%, besonders bevorzugt etwa 0,01 bis etwa 40 Gew.-% und insbesondere etwa 0,1 bis etwa 30 Gew.-% enthalten.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl und dergleichen in Frage.

### Verfahren und Verwendungen

Die Erfindung umfasst des Weiteren die Verwendung von Verbindungen der Formel (I)
- zur Verstärkung der Kühlwirkung, der Frische und des Impacts von physiologischen Kühlwirkstoffen,
- zur Verringerung der Bitterkeit von physiologischen Kühlwirkstoffen

In gleicher Weise beansprucht wird die Verwendung der erfindungsgemäßen Mischungen zur Verstärkung oder / und zur Darstellung des von kosmetischen Zubereitungen, Mundpflegemitteln, pharmazeutischen Zubereitungen, Nahrungsmitteln sowie Aroma- oder Parfümzubereitungen.

Ein letzter Gegenstand der Erfindung betrifft Arzneimittel, enthaltend die erfindungsgemäßen Mischungen zur Befreiung der Atemwege.

### Beispiele

### Herstellbeispiel H1

### Herstellung von 2-Phenyl-but-2-enal

51 g Natriumacetat wurden in 100 g Wasser gelöst und 100 g Ethanol zugegeben. Bei 15°C wurden 120 g Phenylacetaldehyd und 61,7 g Acetaldehyd innerhalb 1 Stunde parallel zudosiert. Es wurde zunächst 3,5 Stunden bei Raumtemperatur und anschließend 13 Stunden unter Rückfluss gerührt. Nach Zugabe von 500 g Wasser wurden die Phasen getrennt. Die wässrige Phase wurde mit Methyl-tert.-butylether extrahiert und die vereinigten organischen Phasen mit Wasser gewaschen. Nach dem Abdestillieren des Lösungsmittels erhielt man 137 g Rohprodukt. Dieses wurde bei einer Manteltemperatur von 120°C und einem Vakuum von 2 mbar am Dünnschichtverdampfer destilliert, wobei 74 g Destillat anfielen. Das Destillat wurde zur Abtrennung von nicht umgesetztem Phenylacetaldehyd einer weitzeren Destillation an einer 10cm-Füllkörperkolonne unterworfen. Es verblieben 65,5 g Material, das 94,3 % trans- und 3,4% cis-Produktisomeres enthielt.

### Beispiel 1

### Paneluntersuchung von Fondantproben

11 sensorisch geschulte Probanden waren aufgefordert die zur Beschreibung der Proben notwendigen Deskriptoren zu sammeln und in ihrer Intensitätswahrnehmung zu charakterisieren. Abgefragt wurden die unten im Netzdiagramm aufgeführten Deskriptoren (Skala von 0 bis 10).

Eine Probe enthielt ein Standardfondant mit einem Mentholgehalt von 0,05 Gew-% ohne 2-Phenyl-but-2-enal und die Vergleichsprobe enthielt 0,05 Gew-% Menthol sowie zusätzlich 100ppm 2-Phenyl-but-2-enal.

Die erfindungsgemäße Verbindung verändert demnach nicht das erwünschte Geschmacksprofil des Aromas; vielmehr verstärkt es die erwünschten Geschmackseindrücke. Die Intensität von Menthol, Kühle und Impact [Angeschmack, Geschmacksbeginn] wird verstärkt und die Gesamtwirkung des Aromas entfaltet sich besser (Deskriptoren Menthol, Kühlend und Impact). Die Ergebnisse der sensorischen Beurteilung sind in Tabelle 1 zusammengefasst.

**Tabelle 1**

| Sensorische Beurteilung | | |
|---|---|---|
| | **Fondant mit 0,05 Gew-% Menthol** | **Fondant mit 0,05 Gew-% Menthol und 100 ppm 2-Phenyl-but-2-enal** |
| Kühlwirkung | 5,5 | 7,3 |
| Impact | 4,1 | 6,0 |
| Menthol | 5,8 | 7,8 |

### Beispiel 2

Flavour- und Aromakompositionen sowie Formulierungsbeispiele

**Tabelle 2.1**

| Aroma Pfefferminztyp (Angaben in Gew.-%) | |
|---|---|
| **Bestandteil** | **Anteil** |
| 2-Phenyl-but-2-enal | 2 |
| Anethol | 9 |
| I-Menthol (natürlich oder synthetisch) | 35 |
| Pfefferminzöl Piperita Typ (natürlich oder rekonstituiert) | 20 |
| Pfefferminzöl Arvensis Typ (natürlich oder rekonstituiert) | 30 |
| SUMME | 100 |

### Beispiel 2.2

Aroma Pfefferminztyp Cool (angaben in Gew.-%)

| **Bestandteil** | **Anteil [%]** |
|---|---|
| 2-Phenyl-but-2-enal | 4 |
| Anethol | 9 |
| I-Menthol (natürlich oder synthetisch) | 35 |
| Pfefferminzöl Piperita Typ (natürlich oder rekonstituiert) | 20 |
| Pfefferminzöl Arvensis Typ (natürlich oder rekonstituiert) | 20 |
| 2-Isopropyl-N,2,3-trimethylbutyramide (WS-23) | 2 |
| (1*R*,2*S*,5*R*)-N-Ethyl-2-isopropyl-5-methylcyclohexanecarboxamide (WS-3) | 2 |
| Menthol-propyleneglycol-carbonate (Frescolat MPC^{®}) | 2 |
| Menthol-ethylenglycol-carbonate (Frescolat MGC^{®}) | 2 |
| I-Menthyl lactat (Frescolat ML^{®}) | 2 |
| SUMME | 100 |

### Beispiel 2.3

Aroma Krauseminztyp (Angaben in Gew.-%)

| **Bestandteil** | **Anteil [%]** |
|---|---|
| 2-Phenyl-but-2-enal | 1 |
| Anethol | 9 |
| I-Menthol (natürlich oder synthetisch) | 30 |
| Pfefferminzöl Piperita Typ (natürlich oder rekonstituiert) | 5 |
| Pfefferminzöl Arvensis Typ (natürlich oder rekonstituiert) | 5 |
| I-Carvon | 15 |
| Krauseminzöl Cardiaca Typ (natürlich oder rekonstituiert) | 15 |
| Krauseminzöl spicata Typ (natürlich oder rekonstituiert) | 15 |
| SUMME | 100 |

### Beispiel 2.4

Aroma Wintergrüntyp (Angaben in Gew.-%)

| **Bestandteil** | **Anteil [%]** |
|---|---|
| 2-Phenyl-but-2-enal | 3 |
| Anethol | 9 |
| I-Menthol (natürlich oder synthetisch) | 45 |
| Pfefferminzöl Piperita Typ (natürlich oder rekonstituiert) | 2 |
| Pfefferminzöl Arvensis Typ (natürlich oder rekonstituiert) | 3 |
| Krauseminzöl Spicata Typ (natürlich oder rekonstituiert) | 1 |
| Eugenol | 7 |
| Eucalyptol | 5 |
| Methylsalicylat | 20 |
| SUMME | 100 |

### Beispiel 2.5

Aroma Wintergrüntyp Cool

| **Bestandteil** | **Anteil [%]** |
|---|---|
| 2-Phenyl-but-2-enal | 5 |
| Anethol | 9 |
| I-Menthol (natürlich oder synthetisch) | 40 |
| Pfefferminzöl Piperita Typ (natürlich oder rekonstituiert) | 2 |
| Pfefferminzöl Arvensis Typ (natürlich oder rekonstituiert) | 3 |
| Krauseminzöl Spicata Typ (natürlich oder rekonstituiert) | 1 |
| Eugenol | 7 |
| Eucalyptol | 5 |
| Methylsalicylat | 16 |
| 2-Isopropyl-N,2,3-trimethylbutyramide (WS-23) | 2 |
| (1*R*,2*S*,5*R*)-N-Ethyl-2-isopropyl-5-methylcyclohexanecarboxamide (WS-3) | 2 |
| Menthol-propyleneglycol-carbonate (Frescolat MPC^{®}) | 2 |
| Menthol-ethylenglycol-carbonate (Frescolat MGC^{®}) | 2 |
| I-Menthyl lactat (Frescolat ML^{®}) | 2 |
| (1*R*,2*S*,5*R*)-N-(4-Methoxyphenyl)-2-isopropyl-5-methylcyclohexanecarboxamide (WS-12) | 1 |
| SUMME | 100 |

### Beispiel 2.6

Aroma Eukalyptustyp (Angaben in Gew.-%)

| **Bestandteil** | **Anteil [%]** |
|---|---|
| 2-Phenyl-but-2-enal | 2 |
| Anethol | 18 |
| Eucalyptol | 15 |
| Eucalyptusöl | 5 |
| I-Menthol (natürlich oder synthetisch) | 50 |
| Pfefferminzöl Piperita Typ (natürlich oder rekonstituiert) | 2 |
| Pfefferminzöl Arvensis Typ (natürlich oder rekonstituiert) | 3 |
| SUMME | 100 |

### Beispiel 2.7

Aroma Eukalyptustyp Cool (Angaben in Gew.-%)

| **Bestandteil** | **Anteil [%]** |
|---|---|
| 2-Phenyl-but-2-enal | 4 |
| Mentholmethylether | 4 |
| Anethol | 18 |
| Eucalyptol | 15 |
| Eucalyptusöl | 5 |
| I-Menthol (natürlich oder synthetisch) | 44 |
| Pfefferminzöl Piperita Typ (natürlich oder rekonstituiert) | 2 |
| Pfefferminzöl Arvensis Typ (natürlich oder rekonstituiert) | 3 |
| Menthoneglycerinacetal (Frescolat MGA^{®}) | 5 |
| SUMME | 100 |

### Beispiel 2.8

Aroma Zimttyp (Angaben in Gew.-%)

| **Bestandteil** | **Anteil [%]** |
|---|---|
| 2-Phenyl-but-2-enal | 0,5 |
| Zimtaldehyd | 10 |
| Anethol | 9 |
| Eugenol | 2 |
| I-Menthol (natürlich oder synthetisch) | 40 |
| Pfefferminzöl Piperita Typ (natürlich oder rekonstituiert) | 10 |
| Pfefferminzöl Arvensis Typ (natürlich oder rekonstituiert) | 15 |
| Krauseminzöl Spicata Typ (natürlich oder rekonstituiert) | 8 |
| SUMME | 100 |

### Beispiel 2.9

Aroma Zimttyp Cool (Angaben in Gew.-%)

| **Bestandteil** | **Anteil [%]** |
|---|---|
| 2-Phenyl-but-2-enal | 2 |
| Menthylmethylether | 3 |
| Zimtaldehyd | 10 |
| Anethol | 9 |
| Eugenol | 2 |
| I-Menthol (natürlich oder synthetisch) | 40 |
| Pfefferminzöl Piperita Typ (natürlich oder rekonstituiert) | 10 |
| Pfefferminzöl Arvensis Typ (natürlich oder rekonstituiert) | 10 |
| Krauseminzöl Spicata Typ (natürlich oder rekonstituiert) | 8 |
| (1*R*,2S,5*R*)-N-Ethyl-2-isopropyl-5-methylcyclohexanecarboxamide (WS-3) | 2 |
| (1*R*,2*S*,5*R*)-N-[4-(cyanomethyl)phenyl]-2-isopropyl-5-methylcyclohexanecarboxamide | 0.5 |
| (1*R*,2*S*,5*R*)-N-[2-(pyridin-2-yl)-ethyl]-2-isopropyl-5-methylcyclohexanecarboxamide | 0.5 |
| Menthoneglycerinacetal (Frescolat MGA^{®}) | 1 |
| Menthol-propyleneglycol-carbonate (Frescolat MPC^{®}) | 1 |
| SUMME | 100 |

Diese Aromakompositionen werden in der Regel in den folgenden Konzentrationen in entsprechende Anwendungsbeispiele eingearbeitet:

| | |
|---|---|
| Zahnpasta: | 0.8 - 1.5 Gew.-% |
| Mundwasser: | 0.1 - 0.25 Gew.-% |
| Mundwasserkonzentrat: | 1 - 4 Gew.-% |
| Kaugummi: | 1 - 2 Gew.-% |
| Candies: | 0.1 - 0.3 Gew.-% |

### Anwendungsbeispiele für oben genannte Flavourkompositionen

### Beispiel 2.10

Zuckerfreier Kaugummi mit Aroma Zimttyp Cool (Angaben in Gew.-%)

| **Bestandteil** | **Anteil** |
|---|---|
| Gum Base | 30,00 |
| Sorbit gepulvert | 40,00 |
| Isomalt gepulvert | 9,50 |
| Xylitol | 2,00 |
| Mannit D | 3,00 |
| Aspartame | 0,10 |
| Acesulfam K | 0,10 |
| Emulgum / Plasticizing agent | 0,30 |
| Sorbitol (70% in Wasser) | 13,00 |
| Glycerin | 1,00 |
| Aroma Zimttyp Cool | 1,00 |
| SUMME | 100 |

Bei einer Dosierung von 1% der Aromakomposition im Kaugummi bewirkt das 2-Phenyl-but-2-enal einen deutlichen "Boosteffekt" und vermittelt - im Vergleich zu dem nicht erfindungsgemäßen Aroma - ein verstärktes Frischegefühl zusammen mit einer höheren Mentholgeschmacks- und Kühlwahrnehmung, insbesondere in der Anfangsphase des Verzehrs.

### Beispiel 2.11

Standard Kaugummi mit Aroma Pfefferminztyp (Angaben in Gew.-%)

| **Bestandteil** | **Anteil [%]** |
|---|---|
| Gum Base | 21,00 |
| Glucosesirup | 16,50 |
| Glycerin | 0,50 |
| Zucker gepulvert | 60,00 |
| Aroma Pfefferminztyp | 2,00 |
| SUMME | 100 |

Bei einer Dosierung von 2% der Aromakomposition im Kaugummi bewirkt das 2-Phenyl-but-2-enal ein verstärktes Frischegefühl zusammen mit einer höheren Mentholgeschmacks- und Kühlwahrnehmung und vermittelt - im Vergleich zu dem nicht erfindungsgemäßen Aroma - eine größere Mundfülle, insbesondere beim Kaubeginn.

### Beispiel 2.12

Zahnpasta (Phosphatbase) mit Aroma Krauseminztyp (Angaben in Gew.-%)

| **Bestandteil** | **Anteil** |
|---|---|
| Entionisiertes Wasser | 36,39 |
| Glycerin | 20,00 |
| Solbrol M (Natriumsalz) | 0,15 |
| Sodiummonofluorphosphate | 0,76 |
| Saccharin | 0,20 |
| Dicalciumphosphate-Dihydrate | 36,00 |
| Aerosil 200 | 3,00 |
| Sodiumcarboxymethylcellulose | 1,20 |
| Sodiumlaurylsulfate (Texapon) | 1,30 |
| Aroma Krauseminztyp | 1,00 |
| SUMME | 100 |

Bei einer Dosierung von 1% der Aromakomposition in der Zahnpasta bewirkt das 2-Phenyl-but-2-enal beim Bürsten eine Wirkungsverstärkung des Aromas und vermittelt - im Vergleich zu dem nicht erfindungsgemäßen Aroma - ein deutliches Frischegefühl zusammen mit einer höheren Mentholgeschmacks- und Kühlwahrnehmung, insbesondere beim anfänglichen Bürsten.

### Beispiel 2.13

Zahnpasta (transparente Gelformulierung) mit Aroma Zimttyp (Angaben in Gew.-%)

| **Bestandteil** | **Enteil** |
|---|---|
| Sorbitol 70% | 63,00 |
| Entionisiertes Wasser | 11,31 |
| Saccharin | 0,20 |
| Sodiummonofluorphosphate | 1,14 |
| Solbrol | 0,15 |
| Trinatriumphosphat | 0,10 |
| PEG 1500 (PEG 32) | 5,00 |
| Sident 9 (Abrasive Silica) | 8,00 |
| Sident 22 S (Dickende Silica) | 8,00 |
| Natriumcarboxymethylcellulose | 0,60 |
| Natriumlaurylsulfat | 1,50 |
| Aroma Zimttyp | 1,00 |
| SUMME | 100 |

Bei einer Dosierung von 1% der Aromakomposition in der Zahnpasta gewinnt die Paste beim Bürsten durch das 2-Phenyl-but-2-enal ein klareres Frischeprofil zusammen mit einer höheren Mentholgeschmacks- und Kühlwahrnehmung und vermittelt - im Vergleich zu dem nicht erfindungsgemäßen Aroma - ein eindeutiges retronasales Cooling.

### Beispiel 2.14

Zahnpasta ('Silica opaque') mit Aroma Pfefferminztyp Cool (Angaben in Gew.-%)

| **Bestandteil** | **Anteil** |
|---|---|
| Entionisiertes Wasser | 26,53 |
| Sorbitol 70% | 45,00 |
| Solbrol M Na-Salz | 0,15 |
| Trinatriumphosphat | 0,10 |
| Saccharin | 0,20 |
| Natriummonofluorphosphat | 1,12 |
| PEG 1500 | 5,00 |
| Sident 9 (Abrasive Silica) | 10,00 |
| Sident 22 S (Dickende Silica) | 8,00 |
| Natriumcarboxymethylcellulose | 0,90 |
| Titan (IV) oxid | 0,50 |
| Natriumlaurylsulfat (SLS) | 1,50 |
| Aroma Pfefferminztyp Cool | 1,00 |
| SUMME | 100 |

Bei einer Dosierung von 1% der Aromakomposition in der Zahnpasta gewinnt die Paste beim Bürsten durch das 2-Phenyl-but-2-enal an Mundfülle und erreicht - im Vergleich zu dem nicht erfindungsgemäßen Aroma - ein frischeres Mundgefühl zusammen mit einer höheren Mentholgeschmacks- und Kühlwahrnehmung.

### Beispiel 2.15

Zahnpasta (Calciumcarbonat-Base) mit Aroma Eucalyptustyp Cool (Angaben in Gew.-%)

| **Bestandteil** | **Anteil** |
|---|---|
| Entionisiertes Wasser | 27,50 |
| Saccharin | 0,20 |
| Solbrol M Natriumsalz | 0,20 |
| Natriummonofluorphosphat | 0,80 |
| Sorbitol 70% | 29,00 |
| Calciumcarbonat | 35,00 |
| Sident 22 S (Dickende Silica) | 2,50 |
| Natriumcarboxymethylcellulose | 1,30 |
| Titan (IV) oxid | 0,50 |
| Natriumlaurylsulfat | 2,00 |
| Aroma Eucalyptustyp Cool | 1,00 |
| SUMME | 100 |

Bei einer Dosierung von 1% der Aromakomposition in dieser Zahnpasta verbessert sich - im Vergleich zu dem nicht erfindungsgemäßen Aroma - das sensorische Profil deutlich und der Gesamteindruck überzeugt durch vermehrte Frische zusammen mit einer höheren Mentholgeschmacks- und Kühlwahrnehmung.

### Beispiel 2.16

Mundwasserkonzentrat mit Aroma Wintergrüntyp (Angaben in Gew.-%)

| **Bestandteil** | **Anteil** |
|---|---|
| Ethylalkohol 96% | 42,00 |
| Cremophor RH 455 | 5,00 |
| Entionisiertes Wasser | 48,67 |
| Allantoin | 0,20 |
| Natriumsaccharin 450 | 0,10 |
| Colour L-Blue 5000 (1% in Wasser) | 0,03 |
| Aroma Wintergrüntyp | 4,00 |
| SUMME | 100 |

Bei einer Dosierung von 4% der Aromakomposition in diesem Mundwasser verbessert sich - im Vergleich zu dem nicht erfindungsgemäßen Aroma - der Geschmack überzeugend durch eine stärkere und schnellere Kühlung im Mundraum.

### Beispiel 2.17

Mundwasser ('ready to use' ohne Alkohol) mit Aroma Eukayptoltyp (Angaben in Gew.-%)

| **Bestandteil** | **Anteil** |
|---|---|
| Cremophor RH 455 | 1,80 |
| Entionisiertes Wasser | 87,57 |
| Sorbitol 70% | 10,00 |
| Natriumfluorid | 0,18 |
| Natriumsaccharin 450 | 0,10 |
| Solbrol M Natriumsalz | 0,15 |
| Aroma Eukalyptoltyp | 0,2 |
| SUMME | 100 |

### Beispiel 2.18

Mundwasser ('ready to use' mit Alkohol) mit Aroma Wintergrüntyp Cool (Angaben in Gew.-%)

| **Bestandteil** | **Anteil** |
|---|---|
| Ethylalkohol 96% | 10,00 |
| Cremophor CO 40 | 1,00 |
| Benzoesäure | 0,12 |
| Entionisiertes Wasser | 83,46 |
| Sorbitol 70% | 5,00 |
| Natriumsaccharin 450 | 0,07 |
| L-Blue 5000 (1% in Wasser) | 0,10 |
| Aroma Wintergrüntyp Cool | 0,25 |
| SUMME | 100 |

### Beispiel 2.19

Bonbon ('Hardboiled candy') zuckerfrei mit Aroma Pfefferminztyp (Angaben in Gew.-%)

| **Bestandteil** | **Anteil** |
|---|---|
| Wasser | 2,24 |
| Isomalt | 94,98 |
| Xylitol | 2,40 |
| Sucralose | 0,03 |
| Acesulfame K | 0,050 |
| Zitronensäure | 0,050 |
| Aroma Pfefferminztyp | 0,25 |
| SUMME | 100 |

### Beispiel 2.20

Bonbon ('Hardboiled candy') mit Aroma Krauseminztyp (Angaben in Gew.-%)

| **Bestandteil** | **Anteil** |
|---|---|
| Wasser | 2.75 |
| Zucker | 60,1 |
| Glucosesirup | 36,9 |
| Aroma Krauseminztyp | 0,25 |
| SUMME | 100 |

### Beispiel 2.21

Zuckerfreies Kaugummi (Angaben in gew.-%)

| **Ingredients** | **Amount** |
|---|---|
| Kaugummibasis | 30.00 |
| Sorbitol, gepulvert | Ad 100 |
| Palatinit | 9.50 |
| Xylitol | 2.00 |
| Mannitol | 3.00 |
| Aspartam | 0.10 |
| Acesulfam K | 0.10 |
| Emulgator | 0.30 |
| Sorbitol 70 %. in Wasser | 14.00 |
| Glycerin | 1.00 |
| Aroma Wintergrüntyp Cool | 1.80 |
| SUMME | 100 |

### Beispiel 3

Mundwasser

| **Bestandteil** | **3a)** | **3b)** | **3c)** | **3d)** |
|---|---|---|---|---|
| Ethanol 96% | 42,00 | 42,00 | 30,00 | 30,00 |
| Cremophor RH 455 | 5,00 | 5,00 | 5,00 | 5,00 |
| Entionisiertes Wasser | 51,67 | 51,67 | 63,67 | 63,67 |
| Allantoin | 0,20 | 0,20 | 0,20 | 0,20 |
| Natriumsaccharin 450 | 0,10 | 0,10 | 0,10 | 0,10 |
| Colour L-Blue 5000 (1% in Wasser) | 0,03 | 0,03 | 0,03 | 0,03 |
| Aroma Typ Pfefferminz aus Beispiel 2.2 mit bzw. ohne 2-Phenyl-but-2-enal (der fehlende Anteil in den Rezepturen 3a und c wurde durch Wasser ersetzt) | 1,00 (enthaltend kein 2-Phenyl-but-2-enal) | 1,00 (enthaltend 2-Phenyl-but-2-enal) | 1,00 (enthaltend 2-Phenyl-but-2-enal) | 1,00 (enthaltend kein 2-Phenyl-but-2-enal) |
| SUMME | 100 | 100 | 100 | 100 |
| Intensität des Ethanolgeschmacks | 4,9 | 7,6 | 5,2 | 3,1 |

Ein geschultes Panel bewertete die Intensität des Ethanolgeschmacks auf einer Skala von 1bis 10 (0 =kein Ethanolgeschmack, 10 = sehr starker Ethanolgeschmack). Beispiel 3b verdeutlicht, dass der Zusatz von 2-Phenyl-but-2-enal zu Beispiel 3 a eine deutliche Steigerung der Intensität des Ethanolgeschmacks bewirkt. Beispiel 3 c verdeutlicht, dass der Zusatz von 2-Phenyl-but-2-enal bei gleichzeitiger Verringerung des Ethanolanteils dazu führt, dass dennoch eine annähernd vergleichbar hohe Ethanolintensität wie in Beispiel 3 a wahrgenommen wird.

## Patentansprüche

1. Mischungen, enthaltend
(a) mindestens ein Phenylalkenal der Formel (I) 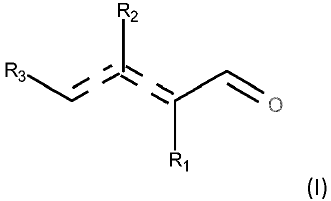 in der R₁ und R₂ unabhängig voneinander Wasserstoff, einen Methyl- oder Phenylrest darstellen, R₃ für Wasserstoff, einen Phenyl-, einen Alkenyl- oder einen linearen oder verzweigten Alkylrest mit 1 bis 5 C-Atomen stehen, und die gestrichelten Doppellinien unabhängig voneinander eine Einfachbindung oder Doppelbindung darstellen, und
(b) mindestens einen physiologischen Kühlwirkstoff.

2. Mischungen nach Anspruch 1 **dadurch gekennzeichnet, dass** in Formel (I) R₁ und R₂ verschieden sind und Wasserstoff oder Phenyl bedeuten, R₃ für Wasserstoff, eine Alkenyl- oder eine lineare oder verzweigte Alkylgruppe mit 1 bis 5 C-Atomen steht, und die gestrichelten Doppellinien unabhängig voneinander eine Einfachbindung oder Doppelbindung darstellen.

3. Mischungen nach den Ansprüchen 1 und/oder 2, **dadurch gekennzeichnet, dass** sie Phenylalkenale (Komponente a) enthalten, die ausgewählt sind aus der Gruppe, die gebildet wird von 2-Phenyl-but-2-enal, 2-Phenyl-pent-2-enal, 3-Phenyl-pent-4-enal, 2-Phenyl-pent-4-enal und deren Mischungen.

4. Mischungen nach mindestens einem der Ansprüche 1 bis 3, **dadurch gekennzeichnet, dass** sie physiologischen Kühlwirkstoffe (Komponente b) enthalten, die ausgewählt sind aus der Gruppe, die gebildet wird von Menthol, L-Menthol, Menthonglycerinacetal, Menthyllactat, substituierten Menthan-3-carbonsäureamiden, Menthan-3-carbonsäure-N-ethylamid, 2-Isopropyl-N,2,3-trimethylbutanamid, substituierten Cyclohexancarbonsäureamiden, 3-Menthoxy-1,2-propandiol, 2-Hydroxyethylmenthylcarbonat, 2-Hydroxypropylmenthylcarbonat, N-Acetylglycinmenthylester, Menthylhydroxycarbonsäureestern,. Menthyl-3-hydroxybutyrat, Menthylmonosuccinat, 2-Mercaptocyclodecanon, Menthyl-2-pyrrolidin-5-oncarboxylat und deren Mischungen.

5. Mischungen nach mindestens einem der Ansprüche 1 bis 4, **dadurch gekennzeichnet, dass** sie weiterhin
(c) mindestens einen Aromastoff und/oder
(d) mindestens ein Lösungsmittel
enthalten.

6. Mischungen nach Anspruch 5, **dadurch gekennzeichnet, dass** sie Aromastoffe (Komponente c) enthalten, die ausgewählt sind aus der Gruppe, die gebildet wird von Anethol, Menthon, Isomenthon, Menthylacetat, Menthofuran, Mintlacton, Eucalyptol, Limonen, Eugenol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Hexanol, Hexanal, cis-3-Hexenol, Linalool, alpha-Terpineol, cis und trans Carvylacetat, p-Cymol, Damascenon, Damascone, Rosenoxid, Dimethylsulfid, Fenchol, Acetaldehyddiethylacetal, cis-4-Heptenal, Isobutyraldehyd, Isovaleraldehyd, cis-Jasmon, Anisaldehyd, Methylsalicylat, Myrtenylacetat, 2-Phenylethylalkohol, 2-Phenylethylisobutyrat, 2-Phenylethylisovalerat, Zimtaldehyd, Geraniol, Nerol und deren Mischungen.

7. Mischungen nach den Ansprüchen 5 und/oder 6, **dadurch gekennzeichnet, dass** sie Lösungsmittel (Komponente d) enthalten, die ausgewählt sind aus der Gruppe, die gebildet wird von Ethylalkohol, 1,2-Propylenglycol, Triacetin, Benzylakohol und fetten Ölen und deren Mischungen.

8. Kosmetische Zubereitungen, Mundpflegemittel, pharmazeutische Zubereitungen, Nahrungsmittel, Aroma- oder Parfümzubereitungen enthaltend Mischungen nach mindestens einem der Ansprüche 1 bis 7.

9. Verwendung von Verbindungen der Formel (I) zur Verstärkung der Kühlwirkung, der Frische und des Angeschmacks von physiologischen Kühlwirkstoffen.

10. Verwendung von Verbindungen der Formel (I) zur Verringerung der Bitterkeit, der stechenden Geschmacks oder der Schärfe von physiologischen Kühlwirkstoffen.

11. Arzneimittel, enthaltend eine Mischung nach den Ansprüchen 1 bis 7 zur Befreiung der Atemwege.

## Claims

1. Mixtures containing
(a) at least one phenylalkenal of formula (I) 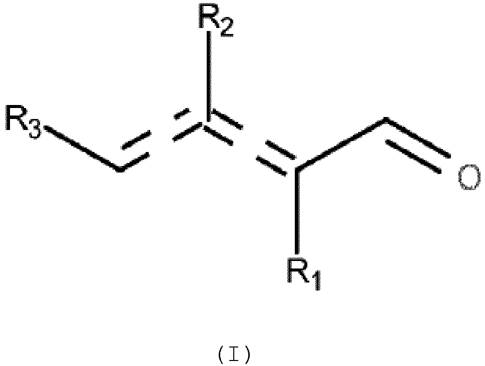 in which R₁ and R₂ independently of one another represent hydrogen, a methyl or phenyl residue, R₃ stands for hydrogen, a phenyl, an alkenyl or a linear or branched alkyl residue with 1 to 5 carbon atoms, and the broken double lines represent, independently of one another, a single bond or a double bond, and
(b) at least one physiological cooling agent.

2. Mixtures according to Claim 1, **characterized in that** in formula (I), R₁ and R₂ are different and denote hydrogen or phenyl, R₃ stands for hydrogen, an alkenyl or a linear or branched alkyl group with 1 to 5 carbon atoms, and the broken double lines represent, independently of one another, a single bond or a double bond.

3. Mixtures according to Claims 1 and/or 2, **characterized in that** they contain phenylalkenals (component a), which are selected from the group comprising 2-phenyl-but-2-enal, 2-phenyl-pent-2-enal, 3-phenyl-pent-4-enal, 2-phenyl-pent-4-enal and mixtures thereof.

4. Mixtures according to at least one of Claims 1 to 3, **characterized in that** they contain physiological cooling agents (component b), which are selected from the group comprising menthol, L-menthol, menthone glycerol acetal, menthyl lactate, substituted menthane-3-carboxylic acid amides, menthane-3-carboxylic acid-N-ethylamide, 2-isopropyl-N,2,3-trimethylbutanamide, substituted cyclohexane-carboxylic acid amides, 3-menthoxy-1,2-propanediol, 2-hydroxyethylmenthyl carbonate, 2-hydroxypropylmenthyl carbonate, N-acetylglycine menthyl esters, menthyl-hydroxy-carboxylic acid esters, menthyl-3-hydroxybutyrate, menthyl monosuccinate, 2-mercaptocyclodecanone, menthyl-2-pyrrolidin-5-one carboxylate and mixtures thereof.

5. Mixtures according to at least one of Claims 1 to 4, **characterized in that** they further contain
(c) at least one aroma substance and/or
(d) at least one solvent.

6. Mixtures according to Claim 5, **characterized in that** they contain aroma substances (component c), which are selected from the group comprising anethole, menthone, isomenthone, menthyl acetate, menthofuran, mint lactone, eucalyptol, limonene, eugenol, pinene, sabinene hydrate, 3-octanol, carvone, gamma-octalactone, gamma-nonalactone, germacren-D, viridiflorol, 1,3E,5Z-undecatriene, isopulegol, piperitone, 2-butanone, ethyl formate, 3-octyl acetate, isoamyl isovalerate, hexanol, hexanal, cis-3-hexenol, linalool, alpha-terpineol, cis and trans carvyl acetate, p-cymene, damascenone, damascone, rose oxide, dimethylsulfide, fenchol, acetaldehyde diethylacetal, cis-4-heptenal, isobutyraldehyde, isovaleraldehyde, cis-jasmone, anisaldehyde, methylsalicylate, myrtenyl acetate, 2-phenylethyl alcohol, 2-phenylethylisobutyrate, 2-phenylethyl isovalerate, cinnamaldehyde, geraniol, nerol and mixtures thereof.

7. Mixtures according to Claims 5 and/or 6, **characterized in that** they contain solvents (component d), which are selected from the group comprising ethanol, 1,2-propylene glycol, triacetin, benzyl alcohol and fatty oils and mixtures thereof.

8. Cosmetic preparations, oral hygiene products, pharmaceutical preparations, foodstuffs, flavoring or perfume preparations containing mixtures according to at least one of Claims 1 to 7.

9. Use of compounds of formula (I) for intensifying the cooling effect, the freshness and the taste of physiological cooling agents.

10. Use of compounds of formula (I) for reducing the bitterness, the burning taste or the pungency of physiological cooling agents.

11. Medicinal product containing a mixture according to Claims 1 to 7 for clearing the respiratory passages.

## Revendications

1. Mélanges, contenant
(a) au moins un phénylalcénal A de formule (I) 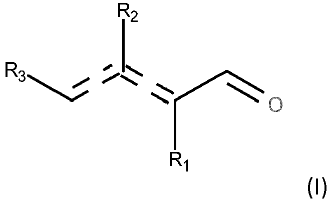 dans laquelle R₁ et R₂ représentent, indépendamment l'un de l'autre, hydrogène, un radical méthyle ou phényle, R₃ représente hydrogène, un radical phényle, un radical alcényle ou un radical alkyle linéaire ou ramifié comprenant 1 à 5 atomes de carbone et les doubles liaisons en pointillés représentent, indépendamment les unes des autres, une simple ou une double liaison, et
(b) au moins une substance active rafraîchissante physiologique.

2. Mélanges selon la revendication 1, **caractérisés en ce que**, dans la formule (I), R₁ et R₂ sont différents et représentent hydrogène ou phényle, R₃ représente hydrogène, un radical alcényle ou un groupe alkyle linéaire ou ramifié comprenant 1 à 5 atomes de carbone et les doubles liaisons en pointillés représentent, indépendamment les unes des autres, une simple ou une double liaison.

3. Mélanges selon les revendications 1 et/ou 2, **caractérisés en ce qu'**ils contiennent des phénylalcénals (composant a) qui sont choisis dans le groupe formé par le 2-phénylbut-2-énal, le 2-phénylpent-2-énal, le 3-phénylpent-4-énal, le 2-phénylpent-4-énal et leurs mélanges.

4. Mélanges selon au moins l'une quelconque des revendications 1 à 3, **caractérisés en ce qu'**ils contiennent des substances actives rafraîchissantes physiologiques (composant b), qui sont choisies dans le groupe formé par le menthol, le L-menthol, le menthone-glycérol-acétal, le lactate de menthyle, les amides substitués de l'acide menthane-3-carboxylique, le N-éthylamide de l'acide menthane-3-carboxylique, le 2-isopropyl-N,2,3-triméthylbutanamide, les amides substitués de l'acide cyclohexanecarboxylique, le 3-menthoxy-1,2-propanediol, le carbonate de 2-hydroxyéthylmenthyle, le carbonate de 2-hydroxypropylmenthyle, l'ester menthylique de la N-acétylglycine, les esters de l'acide menthylhydroxycarboxylique, le 3-hydroxybutyrate de menthyle, le monosuccinate de menthyle, la 2-mercaptocyclodécanone, le 2-pyrrolidin-5-one-carboxylate de menthyle et leurs mélanges.

5. Mélanges selon au moins l'une quelconque des revendications 1 à 4, **caractérisés en ce qu'**ils contiennent en outre
(c) au moins un arôme et/ou
(d) au moins un solvant.

6. Mélanges selon la revendication 5, **caractérisés en ce qu'**ils contiennent des arômes (composant c) qui sont choisis dans le groupe qui est formé par l'anéthol, la menthone, l'isomenthone, l'acétate de menthyle, le menthofurane, la lactone de menthe, l'eucalyptol, le limonène, l'eugénol, le pinène, l'hydrate de sabinène, le 3-octanol, la carvone, la gamma-octalactone, la gamma-nonalactone, le germacrène-D, le viridiflorol, le 1,3E,5Z-undécatriène, l'isopulégol, la pipéritone, la 2-butanone, le formiate d'éthyle, l'acétate de 3-octyle, l'isovalérianate d'isoamyle, l'hexanol, l'hexanal, le cis-3-hexénol, le linalool, l'alpha-terpinéol, l'acétate de cis-carvyle et de trans-carvyle, le p-cymol, la damascenone, la damascone, l'oxyde de rose, le sulfure de diméthyle, le fenchol, le diéthylacétal d'acétaldéhyde, le cis-4-hepténal, l'isobutyraldéhyde, l'isovaléraldéhyde, la cis-jasmone, l'anisaldéhyde, le salicylate de méthyle, l'acétate de myrtényle, l'alcool 2-phényléthylique, l'isobutyrate de 2-phényléthyle, l'isovalérate de 2-phényléthyle, l'aldéhyde cinnamique, le géraniol, le nérol et leurs mélanges.

7. Mélanges selon les revendications 5 et/ou 6, **caractérisés en ce qu'**ils contiennent des solvants (composant d) qui sont choisis dans le groupe formé par l'alcool éthylique, le 1,2-propylèneglycol, la triacétine, l'alcool benzylique et les huiles grasses et leurs mélanges.

8. Préparations cosmétiques, agents de soin de la bouche, préparations pharmaceutiques, aliments, préparations d'arôme ou de parfum contenant des mélanges selon au moins l'une quelconque des revendications 1 à 7.

9. Utilisation de composés de formule (I) pour le renforcement de l'effet rafraîchissant, de la fraîcheur et du goût de substances actives rafraîchissantes physiologiques.

10. Utilisation de composés de formule (I) pour la diminution de l'amertume, du goût piquant ou de l'aigreur de substances actives rafraîchissantes physiologiques.

11. Médicament contenant un mélange selon les revendications 1 à 7 pour la libération des voies respiratoires.
